# EUROPÄISCHE PATENTSCHRIFT

(11) **EP 1 426 808 B1**
(45) Veröffentlichungstag und Bekanntmachung des Hinweises auf die Patenterteilung: **17.09.2008**
(21) Anmeldenummer: 03025245.6
(22) Anmeldetag: 05.11.2003
(51) Int. Cl.: G02B 23/24, A61B 1/00

(54) **Endoskop-Okular**
Endoscope eyepiece
Oculaire d'endoscope

(30) Priorität: 04.12.2002 DE 10256673
(43) Veröffentlichungstag der Anmeldung: 09.06.2004
(73) Patentinhaber: Polydiagnost GmbH, 85276 Pfaffenhofen (DE)
(72) Erfinder: Schaaf, Hansgeorg, Dipl.-Ing., 85293 Reichertshausen (DE)
(74) Vertreter: Nöth, Heinz

(56) Entgegenhaltungen:
- US-A- 4 425 025
- US-A- 5 418 645
- US-A- 5 808 813
- US-A- 6 059 721
- US-A- 6 155 973

## Beschreibung

Die Erfindung betrifft ein Endoskop-Okular nach dem Oberbegriff des Patentanspruches 1.

Ein derartiges Endoskop-Okular ist aus der US-A-6,059,721 bekannt. Beim bekannten Endoskop-Okular ist eine erste zur Scharfeinstellung der Abbildung dienende Linsengruppe in einer ersten Stelleinrichtung in Form eines Tubus gelagert. Eine zweite zur Einstellung der Vergrößerung der Abbildung dienende Linsengruppe ist in einer zweiten Stelleinrichtung gelagert, welche zwei durch Federvorspannung gegeneinander gedrückte Tuben aufweist und gegenüber der ersten Einstelleinrichtung in zwei möglichen Rastpositionen gehalten wird. Mittels eines Einstellrades können die beiden Linsengruppen gleichzeitig axial verstellt werden. Der Abstand der der Vergrößerung dienenden Linsengruppe gegenüber der der Scharfeinstellung dienenden Linsengruppe ist durch die beiden Rastpositionen festgelegt.

Aus US-A-6,155,973 ist ein Endoskop-Okular bekannt, bei dem die Scharfeinstellung und die Einstellung der Vergrößerung der Abbildung mittels separater Einstelleinrichtungen erfolgt. Die Einstellung der Fokussierung erfolgt mittels eines Drehknopfes und die Einstellung der Vergrößerung mittels einer Verstellhülse.

Aufgabe der Erfindung ist es, ein Endoskop-Okular der eingangs genannten Art zu schaffen, welches mittels einfacher Einstellung an verschieden große Bildleitsysteme stufenlos angepasst werden kann.

Diese Aufgabe wird erfindungsgemäß durch die Merkmale des Patentanspruches 1 gelöst.

Bei der Erfindung werden die beiden Linsengruppe in einer Axialführungseinrichtung, insbesondere einer Führungshülse axial geführt und mittels einer drehbar an der Axialführungseinrichtung gelagerten Verstellhülse axial verstellt, wobei beide Linsengruppen sich bewegen und zur Scharfeinstellung der jeweiligen Abbildung bei variabel einstellbaren Vergrößerung der Abstand zwischen den beiden Linsengruppen entsprechend verändert wird.

Das derart ausgebildete Endoskop-Okular kann als Wechselokular für Endoskope mit unterschiedlichen Größen der Bildleitsysteme verwendet werden. Das Wechselokular kann für herkömmliche Bildleitsystemgrößen mit Bildleitern für beispielsweise 1.500 Pixel, 3.000 Pixel, 6.000 Pixel, 10.000 Pixel und 30.000 Pixel verwendet werden. Die Vergrößerung des vom Bildleitsystem gebildeten Zwischenbildes erfolgt mit Hilfe einer einzigen Verstelleinrichtung, die als drehbar gelagerte Verstellhülse ausgebildet ist, wobei automatisch aufgrund des gesteuerten Abstands zwischen den beiden Linsengruppen und dem distalen Endes des Bildleitsystems die Scharfeinstellung der jeweils vergrößerten Abbildung erreicht wird.

Dabei wird vorzugsweise der axiale Abstand einer fest an der ersten Linsengruppe vorgesehenen Apertur gegenüber dem distalen Ende des Bildleitsystems verstellt. Das Okular ist mit dem Endoskop mittels eines lösbaren Verschlusses verbunden. Die beiden Linsengruppen sind an der vorzugsweise als Führungshülse ausgebildeten Axialführungseinrichtung unverdrehbar geführt.

Bei hülsenförmig ausgebildeter Axialführungseinrichtung werden die beiden Linsengruppen innerhalb der Führungshülse axial verschiebbar geführt.

Die Verstelleinrichtung besitzt neben der Verstellhülse noch zwei separat wirkende Stelleinrichtungen, von denen die eine Stelleinrichtung die Drehbewegung der Verstellhülse über ein Schraubengewinde und die andere Stelleinrichtung über eine Kurve auf die jeweils zugeordnete Linsengruppe überträgt und in eine axiale Bewegung der jeweiligen Linsengruppe wandelt. Dabei wird die erste Linsengruppe, welche als Feldlinsenglied dem distalen Ende des Bildleitsystems am nächsten liegt, über die Stelleinrichtung mit dem Gewinde, welches an einer Gewindehülse vorgesehen sein kann, axial bewegt. Gleichzeitig wird die zweite Linsengruppe, welche als Augenlinsenglied von der Stelleinrichtung mit der Kurve, die insbesondere an einer Kurvenhülse vorgesehen ist, axial verschoben. Die aus der Drehung der Verstellhülse abgeleitete axiale Bewegungskomponente wird über Mitnehmer, die vorzugsweise in der Axialführungseinrichtung axial geführt sind, auf die beiden Linsengruppen übertragen.

Hierzu kann das Gewinde der einen Stelleinrichtung in ein Gewinde an der Verstellhülse eingreifen, so dass die Drehbewegung in eine Axialbewegung der Stelleinrichtung umgesetzt wird. Ferner kann die mit der Kurve (Steuerkurve) ausgebildete Stelleinrichtung fest mit der Verstellhülse verbunden sein, so dass die Drehbewegung über die Kurve auf die zugeordnete Linsengruppe übertragen wird und diese Linsengruppe entsprechend axial verschoben wird.

In vorteilhafter Weise kann an einem Bildwiedergabegerät, das über eine Kamera an das Okular angeschlossen ist, die Bildgröße der endoskopischen Abbildung am Bildwiedergabegerät, insbesondere Monitor oder Bildschirm eingestellt werden.

Anhand der Figuren wird an einem Ausführungsbeispiel die Erfindung noch näher erläutert.

Es zeigt
- Fig. 1: einen Längsschnitt durch ein Ausführungsbeispiel eines als Durchsichtokular ausgebildeten Wechselokulars, das ein Ausführungsbeispiel der Erfindung ist;
- Fig. 2: eine schnittbildliche Darstellung einer ersten Linsengruppe des in Fig. 1 dargestellten Ausführungsbeispiels;
- Fig. 3: eine schnittbildliche Darstellung einer zweiten Linsengruppe, welche im Ausführungsbeispiel der Fig. 1 vorhanden ist;
- Fig. 4: in perspektivischer Darstellung eine als Führungshülse ausgebildete Axialführungseinrichtung, welche im Ausführungsbeispiel der Fig. 1 verwendet werden kann;
- Fig. 5: ein Ausführungsbeispiel für eine mit einem Schraubengewinde versehene Gewindehülse für die eine Stelleinrichtung in die beim Ausführungsbeispiel der Fig. 1 verwendet werden kann;
- Fig. 6: eine mit einer Kurve ausgestattete Kurvenhülse für die andere Stelleinrichtung, welche beim Ausführungsbeispiel der Fig. 1 zum Einsatz kommen kann; und
- Fig. 7: eine schematische Darstellung der beiden Linsengruppen und ihre Anordnung gegenüber einem distalen Endstück eines aus einem Bildleitfaserbündel bestehenden Bildleitsystems.

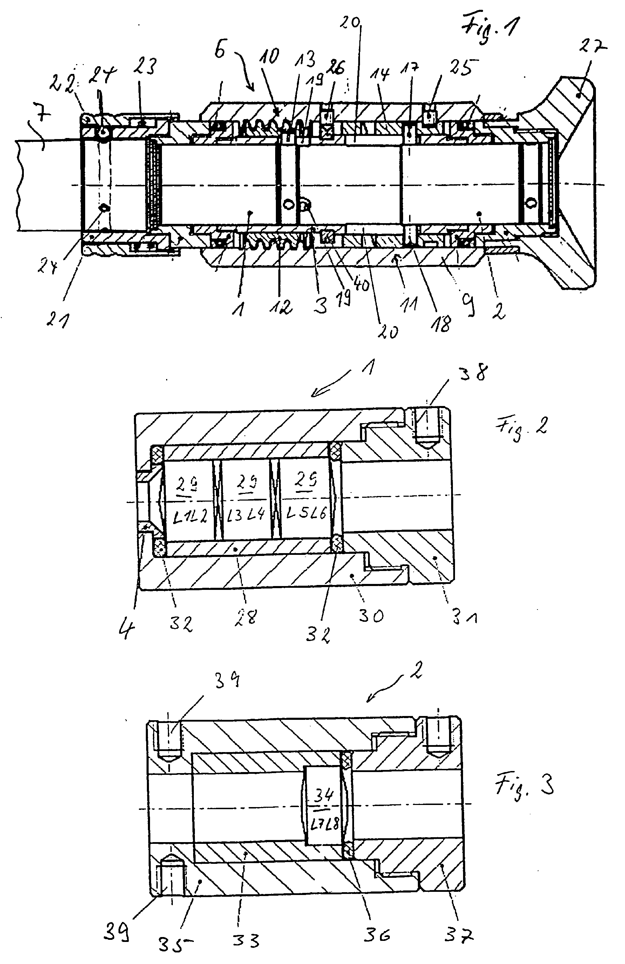

Das in den Figuren dargestellte Endoskop-Okular ist ein Durchsichtokular und kann als Wechselokular an Endoskope mit unterschiedlichen Bildsystemgrößen, beispielsweise mit Bildleitern zwischen 1.500 Pixel und 30.000 Pixel verwendet werden. Zum lösbaren Ankoppeln des Okulars 6 dient ein Verschluss 21, welcher als Kugelschnappverschluss ausgebildet ist. Dieser besitzt eine Schiebehülse 22, die gegen eine Feder 23 verschiebbar am Verschlusskörper gelagert ist. Die Kugeln 24 (beim dargestellten Ausführungsbeispiel drei Kugeln) greifen in eine umlaufende Nut am Endoskopende ein. Zum Lösen wird die Schiebehülse 22 gegen die Kraft der Feder 23 in der Fig. 1 nach rechts verschoben, so dass die Kugeln außer Eingriff mit der umlaufenden Nut kommen und das Okular 6 und das Endoskop 7 voneinander gelöst werden können. Das Okular 6 kann dann mit einem anderen Endoskop, das ein unterschiedliches Bildleitsystem hat verbunden werden.

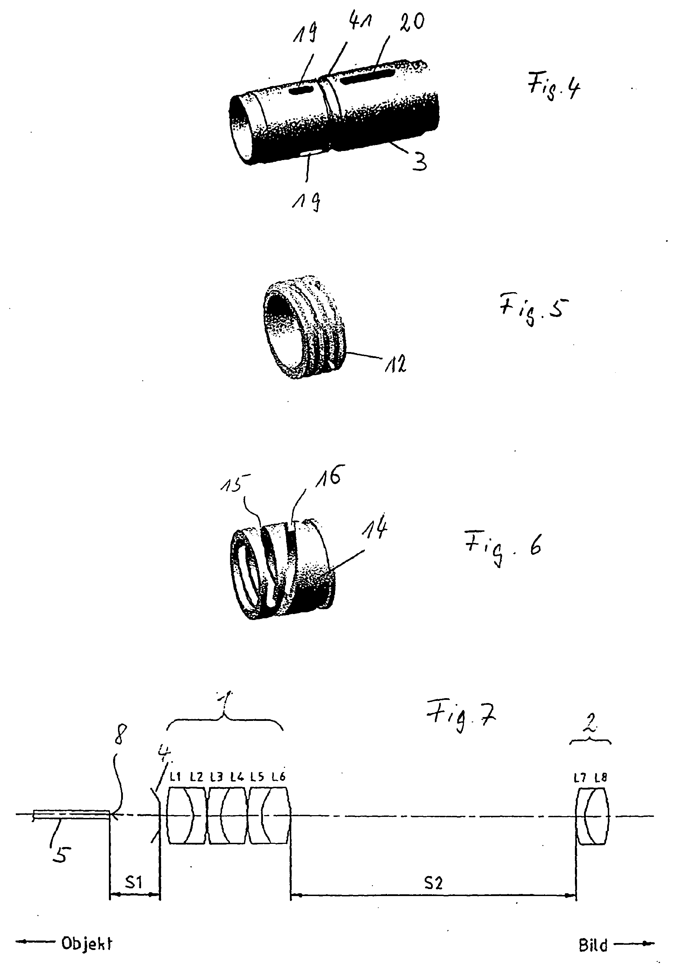

Das Okular 6 besitzt eine Axialführungseinrichtung 3, welche perspektivisch in der Fig. 4 dargestellt ist. Die Axialführungseinrichtung ist als Hülse ausgebildet. In der hülsenförmigen Axialführungseinrichtung 3 befinden sich zwei Linsengruppen, nämlich eine erste Linsengruppe 1, welche als Feldlinse dient, und eine zweite Linsengruppe 2, welche als Augenlinse dient. Die beiden Linsengruppen 1 und 2 sind in der Axialführungseinrichtung 3 in axialer Richtung verschiebbar gelagert.

Die Linsengruppe 1 ist im einzelnen in Fig. 2 dargestellt. Sie besitzt eine Linsenaufnahme 30, an deren vorderem (linken) Ende eine Blende (Apertur) 4 fest eingebaut ist. Ein vorderes Linsenpaar (L1, L2) 29 liegt, wie in der Figur dargestellt ist, am rechten Ende der Blende an. Weitere Linsenpaare (L3, L4 und L5, L6) sind in einer geschlitzten Hülse 28 eingespannt. Hierdurch wird eine definierte Lage der Linsen zueinander und zur Blende 4 erreicht. Auf beiden Seiten sind die Linsenpaare 29 durch O-Ringe 32 abgedichtet. In einem beispielsweise aufgeschraubten Deckel 31 befindet sich eine Durchgangsbohrung mit dem erforderlichen Durchmesser.

In Fig. 3 ist eine Ausführungsform der zweiten Linsengruppe 2 dargestellt. Ein Linsenpaar (L7, L8) 34 ist in einer Spannhülse 33 angeordnet. Die Spannhülse 33 dient gleichzeitig als Abstandhalter zur Einstellung des Abstandes zwischen dem Linsenpaar 34 und der Vorderkante (linke Kante) einer Linsenaufnahme 35. Unter Zwischenlegung eines O-Ringes 36 ist durch beispielsweise Schraubenverbindung ein Deckel 37 fest mit der Linsenaufnahme 35 verbunden. In der Linsenaufnahme 35 der Spannhülse 33 und dem Deckel 37 befinden sich Durchgangsbohrungen mit dem gleichen Durchmesser wie die Durchgangsbohrung im Deckel 31 der Linsengruppe 1.

Die Linsenpaare 29 und 34 bestehen zur Vermeidung von chromatischer Aberration jeweils aus einer konvexen und konkaven Linse.

Für eine variable Größeneinstellung sind die Linsengruppen 1 und 2 axial zueinander verschiebbar. Dabei ist die Linsengruppe zur Schärfeinstellung über einen kürzeren Weg verschiebbar als die Linsengruppe 2, welche zur Einstellung der Vergrößerung dient.

Die gleichzeitige axiale Verschiebung der beiden Linsengruppen 1 und 2 erfolgt mit Hilfe einer einzigen Verstelleinrichtung, welche beiden Linsengruppen zugeordnet ist. Die Verstelleinrichtung ist derart ausgebildet, dass mit Hilfe einer einzigen Verstellbewegung, insbesondere Drehung beide Linsengruppen 1 und 2 gleichzeitig bewegt werden.

Die Verstelleinrichtung besitzt beim dargestellten Ausführungsbeispiel eine Verstellhülse 9, welche drehbar am Okular 6 gelagert ist und an der Außenseite des Okulars 6 angeordnet ist. Die Drehbewegung der Verstellhülse 9 wird über zwei separate Stelleinrichtungen 10 und 11, von denen eine erste Stelleinrichtung 10 der Linsengruppe 1 und eine zweite Stelleinrichtung 11 der Linsengruppe 2 zugeordnet ist, in die jeweiligen axialen Verstellwege für die Linsengruppen 1 und 2 umgewandelt.

Die erste Stelleinrichtung 10, welche der Linsengruppe 1 zugeordnet ist, enthält eine Gewindehülse 12, welche axial verschiebbar auf der Außenseite der hülsenförmigen Axialführungseinrichtung 3 gelagert ist. Die Gewindehülse 12 befindet sich zwischen der Axialführungseinrichtung 3 und der Verstellhülse 9. Drei stiftförmige Mitnehmer 13 ragen durch Langlöcher 19 in der Axialführungseinrichtung 3 und sind fest mit der Linsengruppe 1, beispielsweise durch Schraubenverbindungen in entsprechenden Gewindebohrungen 38 (Fig. 2) verbunden. Ferner sind die stiftförmigen Mitnehmer 13 fest mit der Gewindehülse 12 verbunden. Die Langlöcher 19 in der Axialführungseinrichtung 3 und die stiftförmigen Mitnehmer 13 besitzen Winkelabstände von 120° zueinander. Es kann auch nur ein Mitnehmer 13 vorgesehen sein.

Die Gewindehülse 12 steht mit einem Außengewinde, welches vorzugsweise als Trapezgewinde ausgebildet ist, mit einem angepassten Innengewinde der Verstellhülse 9 in Eingriff. Durch Verdrehen der Verstellhülse 9 wird daher die Gewindehülse 12, welche auf der Außenseite der Axialführungseinrichtung 3 verschiebbar gelagert ist, axial verschoben, wobei diese Verschiebebewegung über den wenigstens einen Mitnehmer 13 auf die Linsengruppe 1 übertragen wird.

Die zweite Stelleinrichtung 11, welche der zweiten Linsengruppe 2 zugeordnet ist, besitzt eine Kurvenhülse 14, welche drehfest mit der Verstellhülse 9 verbunden ist. Hierzu können beispielsweise drei Befestigungsstifte 25, welche im Winkelabstand von 120° angeordnet sind, dienen. Die im Ausführungsbeispiel dargestellte Kurvenhülse besitzt zwei Führungsnuten 15, 16 (Fig. 6). Die Führungsnuten 15, 16, welche als Kurvennuten von der Schraubengeometrie des Trapezgewindes der Gewindehülse 12 abweichen, besitzen einen Winkelabstand von 180° zueinander. In die Führungsnuten 15, 16 greifen zwei diametral an der Linsengruppe 2 in Gewindebohrungen 39 (Fig. 3) eingeschraubte stiftförmige Mitnehmer 17, 18 ein. Die Mitnehmer 17, 18 ragen durch zwei Langlöcher 20, welche ebenfalls einen Winkelabstand von 180° am Umfang der Axialführungseinrichtung 3 aufweisen, hindurch. Damit wird die Drehbewegung der Verstellhülse 9 in eine durch die Führungsnuten 15, 16 bestimmte Axialverschiebung der zweiten Linsengruppe 2 entlang der Axialführungseinrichtung 3 umgewandelt. Anstelle von zwei Führungsnuten kann die Kurvenhülse 14 auch nur eine Führungsnut aufweisen. Durch die beiden Führungsnuten 15, 16 wird jedoch eine verbesserte Genauigkeit der gesteuerten Axialbewegung der Linsengruppe 2 erreicht.

Die Verstellhülse 9 ist gegenüber der Axialführungseinrichtung 3 in axialer Richtung fixiert. Hierzu dient ein Führungsring 40, welcher mit verschraubten Befestigungsstiften 26 fest mit der Verstellhülse 9 verbunden ist. Vorzugsweise werden drei um 120° versetzte Befestigungsstifte 26 verwendet. Der Führungsring 40 ist in einer umlaufenden Nut 41 (Fig. 4) der Axialführungseinrichtung 3 drehbar gelagert.

Durch Verdrehen der Verstellhülse 9 wird der Abstand S1 der Blende (Apertur) 4 und damit der ersten Linsengruppe 1 gegenüber einem distalen Ende 8 des Bildleitsystems 5 des Endoskops 7 verstellt. Gleichzeitig wird der Abstand S2 zwischen den beiden Linsengruppen 1 und 2 verstellt (Fig. 7). Wie aus Fig. 1 zu ersehen ist, befindet sich die Linsengruppe 1 in der Nähe des distalen Endes 8 des Bildleitsystems und dient zur Scharfeinstellung der Abbildung. Die Linsengruppe 2 befindet sich in der Nähe eines Okulartrichters 27 und dient als Augenlinse zur Einstellung der Vergrößerung. Durch Verschieben der beiden Linsengruppen 1 und 2 kann die Vergrößerung mit Hilfe des Okulars 6 um ca. ± 30 % variiert werden. Das Okular ist variabel fokussierbar und kann auf jede gewünschte Größe des Bildleitsystems mit beispielsweise 24- bis 36-fache Vergrößerung eingestellt werden. Auf diese Weise werden die unterschiedlichen Okulare mit fest eingestelltem Fokus, welche bisher für unterschiedliche Bildleitsystemgrößen der Endoskope erforderlich waren, durch ein einziges Okular ersetzt. Durch eine einzige Verstellbewegung, insbesondere Drehung der Verstellhülse 9 erreicht man gleichzeitig die Vergrößerungseinstellung und die Scharfeinstellung des abzubildenden Objekts. Die erste Linsengruppe 1 ist beim Ausführungsbeispiel zur Schärfeeinstellung über einen Weg von 1,7 mm und die zweite Linsengruppe 2 über einen Weg von 8,29 mm axial verschiebbar. Folgende Kombinationen der Abstände S1 und S2 führen zu optimaler Abbildungsgüte.

| | | | | | | | | | |
|---|---|---|---|---|---|---|---|---|---|
| S1 | 4,0 | 4,1 | 4,2 | 4,3 | 4,4 | 4,5 | 4,6 | 4,7 | 4,8 |
| S2 | 33,45 | 32,41 | 31,48 | 30,62 | 29,84 | 29,11 | 28,44 | 27,82 | 27,24 |
| | | | | | | | | | |
| S1 | 4,9 | 5,0 | 5,1 | 5,2 | 5,3 | 5,4 | 5,5 | 5,6 | 5,7 |
| S2 | 26,70 | 26,20 | 25,74 | 25,30 | 24,88 | 24,49 | 24,13 | 23,78 | 23,46 |
| (Angaben in mm) | | | | | | | | | |

Im folgenden werden Abmessungen der Blende und der Linsen sowie der Abstände zwischen den Bauteilen einer bevorzugten Ausführungsform angegeben.

| | Luftabstand/ Mittendicke | Außendurchmesser | freier Durchmesser | Radius |
|---|---|---|---|---|
| Faserbündel | | 0,35-0,8 | 0,8 | |
| Abstand | S1 | | 4,5 | |
| Aperturblende | 0 | | 2,53 | |
| Abstand | 0,7 | | 4,5 | |
| Linse L1 Linse L2 | 2,6 1,30 | 5,5 5,5 | 4,5 4,5 | 12,25/-4,6 -4,6/-13,58 |
| Linse L3 Linse L4 | 1,3 2,60 | 5,5 5,5 | 4,5 4,5 | 13,58/4,6 4,6/-12,25 |
| Linse L5 Linse L6 | 1,3 2,6 | 5,5 5,5 | 4,5 4,5 | 13, 58/4, 6 4,6/-12,25 |
| Abstand | S2 | | 4,5 | |
| Linse L7 Linse L8 | 0,9 2,2 | 5,5 5,5 | 4,5 4,5 | 10, 93/4, 217 4,217/-9,2 |
| (Angaben in mm) | | | | |

### [Bezugszeichenliste]

- 1: erste Linsengruppe
- 2: zweite Linsengruppe
- 3: Axialführungseinrichtung
- 4: Blende (Apertur)
- 5: Bildleitsystem (Bildleiter-Faserbündel)
- 6: Okular
- 7: Endoskop
- 8: distales Ende des Bildleitsystems
- 9: Verstellhülse
- 10: erste Stelleinrichtung
- 11: zweite Stelleinrichtung
- 12: Gewindehülse
- 13: Mitnehmer
- 14: Kurvenhülse
- 15: Führungsnut
- 16: Führungsnut
- 17: Mitnehmer
- 18: Mitnehmer
- 19: Langloch
- 20: Langloch
- 21: Verschluss
- 22: Schiebehülse
- 23: Feder
- 24: Kugeln
- 25: Befestigungsstifte
- 26: Befestigungsstifte
- 27: Okulartrichter
- 28: geschlitzte Hülse
- 29: Linsenpaare
- 30: Linsenaufnahme
- 31: Deckel
- 32: O-Ringe
- 33: Spannhülse
- 34: Linsenpaar
- 35: Linsenaufnahme
- 36: O-Ring
- 37: Deckel
- 38: Gewindebohrung
- 39: Gewindebohrung
- 40: Führungsring
- 41: umlaufende Nut

## Patentansprüche

1. Endoskop-Okular zur Abbildung eines von einem Endoskop-Bildleitsystem übermittelten Objektzwischenbildes mit einer ersten in einer ersten Stelleinrichtung (10) gelagerten Linsengruppe (1) zur Scharfeinstellung der Abbildung, einer zweiten in einer zweiten Stelleinrichtung (11) gelagerten Linsengruppe (2) zum Einstellen der Vergrößerung der Abbildung, einer Axialführungseinrichtung (3), in welcher die beiden Linsengruppen (1, 2) geführt sind, und einer Verstelleinrichtung (9, 12, 14), mit welcher die beiden Linsengruppen (1, 2) gleichzeitig axial verstellbar sind, **dadurch gekennzeichnet, dass** die Verstelleinrichtung (9, 12, 14) eine um die Axialführungseinrichtung (3) drehbar gelagerte Verstellhülse (9) aufweist, deren Drehung über die beiden separat wirkenden Stelleinrichtungen (10, 11) in die gleichzeitigen Axialbewegungen der beiden Linsengruppen (1, 2) gewandelt ist, wobei zur Änderung des Abstandes der ersten Linsengruppe (1) von der zweiten Linsengruppe (2) die erste Stelleinrichtung (10) ein die Drehbewegung der Verstellhülse (9) in die Axialbewegung der ersten Linsengruppe (1) wandelndes Gewinde und die zweite Stelleinrichtung (11) eine vom Gewinde der ersten, Stelleinrichtung (10) abweichende Kurve, welche die Drehbewegung der Verstellhülse (9) in die Axialbewegung der zweiten Linsengruppe (20) wandelt aufweisen.

2. Endoskop-Okular nach Anspruch 1,
**dadurch gekennzeichnet, dass** der axiale Abstand (S1) einer fest an der ersten Linsengruppe (1) vorgesehenen Blende (4) gegenüber dem distalen Ende (8) des Bildleitsystems (5) eines mit dem Okular (6) verbundenen Endoskops (7) verstellbar ist.

3. Endoskop-Okular nach Anspruch 1 oder 2,
**dadurch gekennzeichnet, dass** die beiden Linsengruppen (1, 2) an der Axialführungseinrichtung (3) unverdrehbar geführt sind.

4. Endoskop-Okular nach einem der Ansprüche 1 bis 3,
**dadurch gekennzeichnet, dass** die der ersten Linsengruppe (1) zugeordnete Stelleinrichtung (10) eine Gewindehülse (12) aufweist, welche gegenüber der Verstellhülse (9) und der Axialführungseinrichtung (3) beim Drehen der Verstellhülse (9) axial verschoben ist, wobei diese Axialverschiebung über wenigstens einen Mitnehmer (13) auf die erste Linsengruppe (1) übertragen wird.

5. Endoskop-Okular nach einem der Ansprüche 1 bis 4,
**dadurch gekennzeichnet, dass** die der zweiten Linsengruppe (2) zugeordnete Stelleinrichtung (11) eine mit der Verstellhülse (9) fest verbundene Kurvenhülse (14) aufweist, die wenigstens eine Führungsnut (15, 16) aufweist, in welche wenigstens ein an der zweiten Linsengruppe (2) befestigter Mitnehmer (17, 18) eingreift.

6. Endoskop-Okular nach Anspruche 5,
**dadurch gekennzeichnet, dass** zwei um 180° versetzte Führungsnuten (15, 16) an der Kurvenhülse (14) vorgesehen sind.

7. Endoskop-Okular nach einem der Ansprüche 1 bis 6,
**dadurch gekennzeichnet, dass** die Mitnehmer (13, 17, 18) stiftförmig ausgebildet sind und durch Langlöcher (19, 20) der Axialführungseinrichtung (3) ragen.

8. Endoskop-Okular nach einem der Ansprüche 1 bis 7,
**dadurch gekennzeichnet, dass** die Verstellhülse (9) in axialer Richtung an der Axialführungseinrichtung (3) fixiert ist.

9. Endoskop-Okular nach einem der Ansprüche 1 bis 8,
**dadurch gekennzeichnet, dass** bei einem am Okular (6) über eine Kamera angeschlossenen Bildwiedergabegerät mittels der Verstellhülse (9) die Bildgröße der endoskopischen Abbildung am Bildwiedergabegerät einstellbar ist.

10. Endoskop-Okular nach einem der Ansprüche 1 bis 9,
**dadurch gekennzeichnet, dass** das Okular (6) als Wechselokular ausgebildet ist und mittels eines Verschlusses (21) mit Endoskopen, die unterschiedlich große Bildleitsysteme (5) aufweisen, verbindbar ist.

## Claims

1. An endoscope eyepiece for imaging of an intermediate object image transmitted by an endoscope image guide system, comprising a first lens group (1) mounted in a first positioning device (10) for focusing the imaging, a second lens group (2) mounted in a second positioning device (11) for adjusting the magnification of the imaging, an axial guide device (3) in which the two lens groups (1, 2) are guided, and a displacement device (9, 12, 14) with which the two lens groups (1, 2) are simultaneously axially displaceable, **characterised in that** the displacement device (9, 12, 14) has a displacement sleeve (9) which is mounted rotatably about the axial guide device (3) and the rotation of which is converted into the simultaneous axial movements of the two lens groups (1, 2) by way of the two separately acting positioning devices (10, 11), wherein to alter the spacing of the first lens group (1) from the second lens group (2) the first positioning device (10) has a screwthread for converting the rotary movement of the displacement sleeve (9) into the axial movement of the first lens group (1) and the second positioning device (11) has a cam which deviates from the screwthread of the first positioning device (10) and which converts the rotary movement of the displacement sleeve (9) into the axial movement of the second lens group (20).

2. An endoscope eyepiece according to claim 1 **characterised in that** the axial spacing (S1) of an aperture (4) fixedly provided at the first lens group (1) is adjustable with respect to the distal end (8) of the image guide system (5) of an endoscope (7) connected to the eyepiece (6).

3. An endoscope eyepiece according to claim 1 or claim 2 **characterised in that** the two lens groups (1, 2) are non-rotatably guided at the axial guide device (3).

4. An endoscope eyepiece according to one of claims 1 to 3 **characterised in that** the positioning device (10) associated with the first lens group (1) has a screwthreaded sleeve (12) which is axially displaced with respect to the displacement sleeve (9) and the axial guide device (3) upon rotation of the displacement sleeve (9), wherein said axial displacement is transmitted by way of at least one entrainment portion (13) to the first lens group (1).

5. An endoscope eyepiece according to one of claims 1 to 4 **characterised in that** the positioning device (11) associated with the second lens group (2) has a cam sleeve (14) which is fixedly connected to the displacement sleeve (9) and which has at least one guide groove (15, 16) into which engages at least one entrainment portion (17, 18) fixed to the second lens group (2).

6. An endoscope eyepiece according to claim 5 **characterised in that** there are provided two guide grooves (15, 16) displaced through 180° on the cam sleeve (14).

7. An endoscope eyepiece according to one of claims 1 to 6 **characterised in that** the entrainment portions (13, 17, 18) are of a pin-shaped configuration and project through slots (19, 20) of the axial guide device (3).

8. An endoscope eyepiece according to one of claims 1 to 7 **characterised in that** the displacement sleeve (9) is fixed in the axial direction to the axial guide device (3).

9. An endoscope eyepiece according to one of claims 1 to 8 **characterised in that** in an image reproducing device connected to the eyepiece (6) by way of a camera the image size of the endoscope imaging is adjustable at the image reproducing device by means of the displacement sleeve (9).

10. An endoscope eyepiece according to one of claims 1 to 9 **characterised in that** the eyepiece (6) is in the form of an interchangeable eyepiece and can be connected by means of a fastening means (21) to endoscopes which have image guide systems (5) of differing sizes.

## Revendications

1. Oculaire d'endoscope pour la reproduction d'une image intermédiaire d'objet transmise par un système de circuit vidéo d'endoscope, comprenant un premier groupe de lentilles (1), monté dans un premier dispositif de réglage (10), pour la mise au point de la reproduction, un second groupe de lentilles (2), monté dans un second dispositif de réglage (11), pour régler l'agrandissement de la reproduction, un dispositif de guidage axial (3), dans lequel sont guidés les deux groupes de lentilles (1, 2), et un dispositif de déplacement (9, 12, 14), qui permet de déplacer simultanément dans la direction axiale les deux groupes de lentilles (1, 2), **caractérisé en ce que** le dispositif de déplacement (9, 12, 14) comporte une douille de déplacement (9), montée tournante autour du dispositif de guidage axial (3) et dont la rotation est convertie, par l'intermédiaire des deux dispositifs de réglage (10, 11) agissant séparément, en déplacements axiaux simultanés des deux groupes de lentilles (1, 2), le premier dispositif de réglage (10) comportant, pour modifier l'écartement du premier groupe de lentilles (1) par rapport au second groupe de lentilles (2), un filetage transformant le mouvement de rotation de la douille de déplacement (9) en déplacement axial du premier groupe de lentilles (1), et le second dispositif de réglage (11) comportant une came divergente du filetage du premier dispositif de réglage (10), qui transforme le mouvement de rotation de la douille de déplacement (9) en déplacement axial du second groupe de lentilles (20).

2. Oculaire d'endoscope suivant la revendication 1, **caractérisé par** la possibilité de réglage de l'écartement axial (S1) d'un diaphragme (4), prévu fixement sur le premier groupe de lentilles (1), par rapport à l'extrémité distale (8) du système de circuit vidéo (5) d'un endoscope (7) raccordé à l'oculaire (6).

3. Oculaire d'endoscope suivant l'une des revendications 1 et 2, **caractérisé en ce que** les deux groupes de lentilles (1, 2) sont guidés sans possibilité de rotation sur le dispositif de guidage axial (3).

4. Oculaire d'endoscope suivant l'une des revendications 1 à 3, **caractérisé en ce que** le dispositif de réglage (10), associé au premier groupe de lentilles (1), comporte une douille filetée (12), qui est déplacée dans la direction axiale par rapport à la douille de déplacement (9) et au dispositif de guidage axial (3) lors de la rotation de la douille de déplacement (9), ce déplacement axial étant transmis au premier groupe de lentilles (1) par l'intermédiaire d'au moins un entraîneur (13).

5. Oculaire d'endoscope suivant l'une des revendications 1 à 4, **caractérisé en ce que** le dispositif de réglage (11), associé au second groupe de lentilles (2), comporte un manchon à came (14) assemblé fixement avec la douille de déplacement (9), lequel manchon présente au moins une gorge de guidage (15, 16), dans laquelle s'engage au moins un entraîneur (17, 18) fixé sur le second groupe de lentilles (2).

6. Oculaire d'endoscope suivant la revendication 5, **caractérisé en ce que** deux gorges de guidage (15, 16) en déport de 180° sont prévues sur le manchon à came (14).

7. Oculaire d'endoscope suivant l'une des revendications 1 à 6, **caractérisé en ce que** les entraîneurs (13, 17, 18) sont réalisés en forme de broches et pénètrent au travers de trous oblongs (19, 20) du dispositif de guidage axial (3).

8. Oculaire d'endoscope suivant l'une des revendications 1 à 7, **caractérisé en ce que** la douille de déplacement (9) est fixée dans la direction axiale sur le dispositif de guidage axial (3).

9. Oculaire d'endoscope suivant l'une des revendications 1 à 8, **caractérisé en ce que,** dans un appareil de reproduction d'images raccordé à l'oculaire (6) par l'intermédiaire d'une caméra, le format d'image de la reproduction endoscopique est réglable sur l'appareil de reproduction au moyen de la douille de déplacement (9).

10. Oculaire d'endoscope suivant l'une des revendications 1 à 9, **caractérisé en ce que** l'oculaire (9) est réalisé sous forme d'oculaire interchangeable, et peut être raccordé au moyen d'une fermeture (21) à des endoscopes, qui présentent des systèmes de circuit vidéo (5) différemment grands.
